(19) Europäisches Patentamt / European Patent Office / Office européen des brevets

(11) **EP 3 056 193 A1**

(12) **EUROPEAN PATENT APPLICATION**

(43) Date of publication:
**17.08.2016 Bulletin 2016/33**

(51) Int Cl.:
**A61K 8/36** *(2006.01)*     **A61Q 19/02** *(2006.01)*
**A61K 8/97** *(2006.01)*

(21) Application number: **16155094.2**

(22) Date of filing: **10.02.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **12.02.2015 BR 102015003242**

(71) Applicant: **EMS S.A.**
**CEP-13186-901 Hortolandia, SP (BR)**

(72) Inventors:
• **BARBOSA, Juliana Maria**
**13186-901 Chácara Assay, Hortolândia, São Paulo (BR)**

• **MASIERO, Silvana**
**13186-901 Chácara Assay, Hortolândia, São Paulo (BR)**
• **COVESI, Leticia Khater**
**13186-901 Chácara Assay, Hortolândia, São Paulo (BR)**
• **CEPEDA, Ana Olívia Tiroli**
**13186-901 Chácara Assay, Hortolândia, São Paulo (BR)**
• **PAVANI, Matheus**
**13186-901 Chácara Assay, Hortolândia, São Paulo (BR)**

(74) Representative: **Cabinet Plasseraud**
**66, rue de la Chaussée d'Antin**
**75440 Paris Cedex 09 (FR)**

(54) **COSMETIC COMPOSITION AND USE THEREOF**

(57)    A cosmetic composition and its use as an anti-oxidant and bleaching agent is described. In particular, said composition comprises different active ingredients, among them a botanical extract, and cosmetically acceptable excipients. The active compounds are selected from ferulic acid, olive leaves extract, maslinic acid, Octadecyl Di-t-butyl-4-hydroxyhydrocinnamate and BHT.

**Description**

**FIELD OF THE INVENTION**

The present invention relates to a cosmetic composition and its use as an antioxidant and bleaching agent. In particular, said composition comprises different active ingredients, among them a botanical extract, and cosmetically acceptable excipients.

**BACKGROUND OF THE INVENTION**

The skin is constantly exposed to a variety of damaging agents to its homeostasis. Such agents may originate in the external environment, including solar radiation and air pollutants, and the internal environment, including reactive species, originated during normal metabolism of the body or due to an external disturbance.

Among the reactive compounds, reactive oxygen species (ROS) are cited as the main class of reactive oxygen species, which are part of the superoxide radicals ($O_2^{\bullet-}$), hydroxyl ($^{\bullet}OH$), peroxyl ($RO_2^{\bullet}$) and nitric oxide ($NO^{\bullet}$), as well as ozone ($O_3$), peroxynitrite ($ONOO^-$), hydrogen peroxide ($H_2O_2$) and singlet oxygen ($^1O2$). Such species are usually produced and degraded in aerobic organisms at a rate such that maintenance of physiological concentrations suitable for cell function occurs. This balance is maintained by antioxidants such as vitamins and enzymes, as well as cell repair processes.

However, when reactive oxygen species are present in excessive amounts in the body, defining so-called oxidative stress, it is an imbalance in the rate of ROS generation in relation to common antioxidant defense organisms. The causes are commonly associated with habits considered unhealthy, such as smoking and physical inactivity, as well as the advancement of age when the body's antioxidant mechanisms begin to fail, and also the incidence of radiation and ambient air pollution.

Accordingly, aging of the skin occurs, which may be defined as a rate of decay and / or metabolism efficiency drop resulting from insufficient production of substances / cells for tissue regeneration, or generation of faulty cells during common in vivo processes. It is possible to classify the process of aging in relation to its generating source, which can be intrinsic and extrinsic, wherein the reactive species mentioned above are present in both cases.

Different sources of skin aging result in characteristic dermal structures. While intrinsic aging is identified by a superficial wrinkling, thinning and sagging of the skin, the extrinsic is characterized by the presence of deep wrinkles and bags, dryness and pigmentation irregularities (Emerit, I. "Free Radicals and Aging of the Skin." Birkhauser Basel 1992; 62: 328-341).

As mentioned above, a mechanism for neutralizing free radicals, inhibiting oxidative stress, is the ingestion of vitamins in natural or supplementary foods, especially vitamin C (ascorbic acid) and Vitamin E (tocopherol), which act as antioxidants in the skin. However, controls of the human body on absorption, distribution and metabolism of such compounds limit the amount that can be effectively released to the skin.

In order to resolve such limitation, a solution for the protection against reactive species is the topical application of antioxidants to maintain the balance of reactive species in the skin, and sunscreen compounds to prevent the formation of new reactive species by radiation.

Antioxidant compounds commonly used in the prior art cosmetic compositions include, in addition to the vitamins mentioned above and derivatives thereof, carotenoid and polyphenol compounds. Some examples include caffeic acid, lipoic acid, astaxanthin, catechins, coenzyme Q10 (ubiquinone), genistein, niacinamide, lycopene, resveratrol and vitamin A (retinoic acid, retinal and retinol).

In addition to damage caused to the skin by continuous exposure to external and internal factors which may cause serious health problems such as the formation of cancer, the skin may also be affected by conditions apparently not harmful to health, but of an aesthetic nature that may trigger psychological problems. Among these are cosmetic changes, such as melasma, which in some cases may psychologically compromise the lives of many people because of its appearance in visible places on the skin.

Melasma is a condition characterized by dark spots on the skin, usually on the face. This condition more frequently affects women but can also be found in men. While there is no definite cause for its appearance, it is believed that the use of contraceptives, pregnancy and exposure to the sun may be important factors. In addition, there are studies linking the condition to genetic predisposition (Rathi, S.K. & Achar, A. "Melasma: A Clinical-Epidemiological Study of 312 Cases "Indian J Dermatol. 2011 Jul-Aug; 56 (4): 380-382).

Currently, melasma treatment methods consist of three types of action: laser, peeling, and bleaching compositions (Grimes, P.E. "Melasma. Etiologic and therapeutic considerations "Arch Dermatol. 1995 Dec; 131 (12): 1453-7). While the former involves the use of radiation to reduce contrast of the skin, the second and third consist of the application of desquamative and bleaching substances, respectively.

The use of lasers for the treatment of melasma has the disadvantage that, if applied incorrectly, can lead to increased skin pigmentation, making treatment ineffective. As for peeling, lightening occurs by regeneration of the surface layers of the skin, so that, during treatment, dermal sensitization occurs which only stops after the end of treatment. Both methods require the presence of a professional for application.

On the other hand, bleaching compositions, despite presenting results with longer application times, constitute a less invasive treatment for pigmentation disorders and may be applied by the subject himself affected by the condition.

Currently, several compounds are used to treat skin pigmentation disorders. Among them, two important compounds are described below.

Hydroquinone (1,4-dihydroxybenzene), found in oats and coffee, is referred to as the main active ingredient in the treatment of hyperpigmentation and has been in use for over 50 years. However, due to adverse effects such as exogenous ochronosis and permanent depigmentation under long-term use, the compound was banned for cosmetic use in Europe by the European Committee (24th Directive 2000/6/EC) and its use can only occur under a doctor's prescription. A similar situation occurred in the United States and Japan. In Brazil, despite its use in cosmetics it has not yet been banned by ANVISA, however new registrations of cosmetic compositions containing the compound are no longer accepted.

Kojic acid (5-hydroxy-2-hydroxymethyl-4H-pyran-4-one) is a fungal metabolite obtained from Aspergillus and Penicillium commonly applied in combination with other agents such as glycolic acid. Despite its known effect on melasma, the acid is commonly associated with skin sensitization, contact dermatitis and erythema. A comparative study of the use of kojic acid in relation to hydroquinone in skin lightening is presented by Monteiro, R.C. et al. ("A comparative study of the efficacy of hydroquinone 4% vs 0.75% kojic acid cream in the treatment of facial melasma, "Indian J Dermatol 2013.; 58: 157) showing the superiority of hydroquinone action in the treatment of melasma.

Thus, in front of the above, there still exists in the art, the need for a cosmetic composition that presents good antioxidant potential and effective lightening action without the adverse effects of the solutions listed above.

Thus, it is an objective of the present invention to provide a cosmetic composition that, in addition to providing a protective effect, especially by antioxidant action, presents a homogenizing action on skin with pigmentation damage, exceeding those described in the prior art.

## SUMMARY OF THE INVENTION

As mentioned above, the present invention relates to a cosmetic composition and its use as an antioxidant and as a bleaching agent, preferably as an antioxidant formulation with bleaching action. The said composition comprises various antioxidants and cosmetically acceptable excipients.

The novel composition herein is formulated with at least three active compounds, preferably antioxidants: ferulic acid, maslinic acid and olive leaves extract (Olea europaea) combined with cosmetically acceptable excipients.

In a preferred embodiment, the composition is formulated with four or five antioxidants, wherein the additional antioxidant active compounds combined with the three mentioned above, are selected from Octadecyl Di-t-butyl-4-hydroxyhydrocinnamate and butylated hydroxytoluene (BHT).

In another preferred embodiment, one or more active compounds, preferably the ferulic and maslinic acids are encapsulated in carriers selected from the following groups: inclusion complexes, polymeric particles or lipid carriers, including liposomes, but not limited to the carriers mentioned above, wherein the particle diameters of these forms are in nano- or micrometer scale.

Unexpectedly, the present inventors have noted a synergistic bleaching action between the active ingredients described herein when inserted in a cosmetic formulation. The object of the invention is thus an antioxidant composition having bleaching action.

The features of the invention will be described in more detail in the detailed description of the invention.

## BRIEF DESCRIPTION OF THE FIGURES

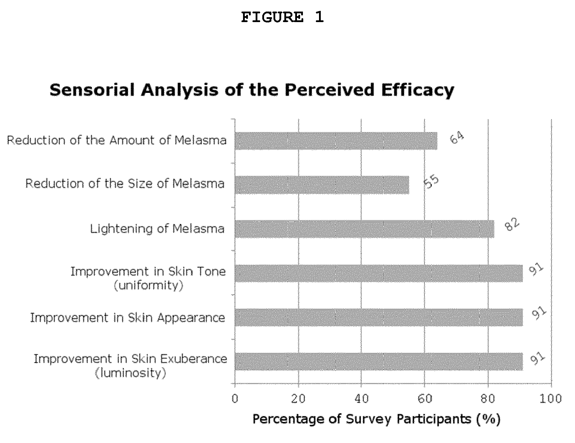

Figure 1 shows the results of analysis of the perceived efficacy of eleven volunteers (n = 11) after 28 days of daily application of the illustrative composition of Example 1.

## DETAILED DESCRIPTION OF THE INVENTION

Presented, in this description, is a cosmetic composition and its use as an antioxidant and a bleaching agent. Said composition comprises the use of a plurality of cosmetically active ingredients with antioxidant activity and cosmetically acceptable excipients.

The composition is formulated with at least three antioxidants, ferulic acid, maslinic acid and olive leaves extract (Olea europaea) combined with cosmetically acceptable excipients, being further selected as optional antioxidants Octadecyl Di-t-butyl-4-hydroxyhydrocinnamate and butylated hydroxytoluene (BHT).

Ferulic acid is a hydroxycinnamic acid found in many vegetables. Currently, it is known to have photo protection action and strong antioxidant activity due to its phenolic group, and can inhibit the formation of melanin and is studied for its possible antitumor action.

Olive leaves (Olea europaea) are known to contain many compounds with antioxidant activity, whose representative compound in greater quantity is oleuropein. This compound has known antioxidant, anti-inflammatory and antimicrobial properties. It is noteworthy that its antioxidant activity was defined as 2.5 times greater than that of vitamin C (ascorbic acid) and E (tocopherol).

Maslinic acid is a naturally occurring triterpene that can be obtained from olive, clove, mint, pomegranate and sage. They are known for their anti-tumor activity, as well as antidiabetic, cardiovascular and neuroprotective, antiparasitic, growth stimulant, antihistamine, anti-inflammatory and antioxidant activity. (Lozano-Mena et al, "Maslinic acid, a natural phytoalexin-type triterpene from olives. - A promising nutraceutical?" Molecules 2014; 19: 11538). Further, its effectiveness in the treatment of erythema and sunburn was reported in a patent application filed by the University of Granada (ES) WO 2008/116959.

During the preparation of a new antioxidant cosmetic composition, the inventors surprisingly identified an unexpected lightening effect of their application on the skin of volunteers with melasma. Such a bleaching effect on the skin is due to the combination of the active ingredients ferulic acid, oleuropein and maslinic acid in certain concentration ranges which act synergistically through their photoprotective, antioxidant and anti-erythema activity in the formation and reduction of melasma. Furthermore, the inventors also identified in a preferred embodiment, that the bleaching effect can be enhanced by an additional antioxidant selected from the substances Octadecyl Di-t-butyl-4-hydroxyhydrocinnamate and BHT.

Octadecyl Di-t-butyl-4-hydroxyhydrocinnamate, as ferulic acid, which is a phenolic antioxidant whose desirability resides in the fact that it is easily soluble in oils and is compatible with surfactants and aqueous systems; a factor that prevents the degradation of antioxidant components and prolongs the lightening efficacy.

Butylated hydroxytoluene (BHT) is a synthetic compound commonly used as an antioxidant in cosmetic and pharmaceutical compositions. It is known that its absorption into the skin is low, so that its action takes place mainly on the surface.

According to the present invention, the terms "cosmetically active ingredient," "active ingredient" and "active" are used interchangeably and refer to compounds in a composition which promotes a desired cosmetic effect.

According to the present invention, the term "cosmetically acceptable" refers to compounds that are commonly used in the cosmetic arts in conjunction with the active ingredients. Particularly, "cosmetically acceptable" refers to compounds which confer, without limitation, the shape, fragrance, stability and coloring of the final composition in a safe and tolerable medium for a user of the final product. In some embodiments a "cosmetically acceptable" component can facilitate absorption of one or more active ingredients in application.

Cosmetically acceptable excipients include, without limitation, pH adjusting agents, conditioners, preservatives, dyes, emollients, emulsifiers, fragrances, thickeners, sequestering agents and vehicles.

Examples of pH adjusting agents include, without limitation, acetic acid, citric acid, hydrochloric acid, lactic acid, sodium bicarbonate, ammonium carbonate, potassium hydroxide, sodium hydroxide and triethanolamine.

Examples of conditioners include, without limitation, copper acetyl methionate, magnesium acetyl methionate, manganese acetyl methionate, zinc acetyl methionate, methylsilanol hydroxyproline aspartate, carnosine and its derivatives, plant extracts such as olive leaves extract (Olea europaea), glutamil aminoethyl imidazole, vegetable oils such as cottonseed oil (Gossypium herbaceum), sunflower seed oil (Helianthus annuus), passion fruit seed oil (Passiflora edulis), and grapeseed oil (Vitis vinifera).

Examples of preservatives include, without limitation, benzoic acid, benzyl alcohol, sodium benzoate, cetylpyridinium chloride, benzalkonium chloride, benzethonium chloride, phenoxyethanol, imidazolidinyl urea, methylchloroisothiazolinone, methylisothiazolinone, parabens and mixtures thereof.

Examples of dyes include, without limitation, CI10315 (yellow), CI12085 (red), CI15510 (orange), CI15800 (red), CI15880 (red), CI15985 (yellow), CI19140 (yellow), CI20170 (brown), CI42053 (green), CI42080 (blue), CI42090 (blue), CI42510 (violet), CI45100 (red), CI45370 (orange), CI59040 (green), CI60725 (violet), CI60730 (violet), CI75170 (white), CI77891 (white), caramel and mixtures thereof.

Examples of emollients include, without limitation, stearic acid, lactic acid, animal fats such as lanolin, vegetable oils such as cottonseed oil (Gossypium herbaceum), sunflower seed oil (Helianthus annuus) passion fruit seed oil (Passiflora edulis), grapeseed oil (Vitis vinifera), propylheptyl caprylate, caprylyl glycol, coco-caprylate / caprate, cyclomethicone, dimethicone, ethoxydiglycol, glycerin, lactose, cetyl palmitate, sorbitol, caprylic capric triglycerides and urea.

Examples of emulsifiers include, without limitation, cetearyl alcohol, cetyl alcohol, stearyl alcohol and mixtures thereof, glyceryl stearate, ethoxylated fatty alcohols, sorbitan esters such as sorbitan monooleate and sorbitan stearate, polysorbates and lecithin.

Examples of thickeners include, without limitation, waxes, such as beeswax, carnauba wax and candelilla wax and lanolin, polysaccharides; among which are starch, gums such as gum arabic, guar gum, xanthan gum, tragacanth, agar agar, alginates and carrageenans; cellulose and its derivatives such as microcrystalline cellulose, cellulose acetate, carboxymethylcellulose and hydroxyethylcellulose, glyceryl stearate, polyethylene glycol, polyvinylpyrrolidone, polyvinyl alcohol, carbopol, polyacrylic acid derivatives and silanes, alkyl polyacrylates, alkyl polymethacrylates and mixtures thereof.

Examples of fragrances include, without limitation, natural and synthetic fragrances and mixtures thereof.

Examples of scavengers include, without limitation, EDTA, disodium EDTA, tetrasodium EDTA, monobasic sodium phosphate and dibasic sodium phosphate and mixtures thereof.

Examples of carriers include, without limitation, water, ester groups, mineral oil and vegetable oils and mixtures thereof when in an emulsified system.

The cosmetic composition may be presented in several forms, including, without limitation, aerosol, cream, gel, gel-cream, lotion or serum, preferably a serum. A person skilled in the art will recognize that the cosmetic medium will be defined by the choice of cosmetically acceptable excipients and absorption / action of various active ingredients which can vary depending on the selected cosmetic form.

As stated earlier, a bleaching action of skin for the antioxidant composition under application was surprisingly detected. It is believed that this fact is due to a synergistic combination of the present active ingredients, particularly ferulic acid, olive leaves extract (Olea europaea) and maslinic acid. It should not be understood, however, that such action is limited to these components.

The concentration ranges for each active ingredient are described below. It is understood that each component present in an amount within a range of concentration may be in combination with any amounts of other components, provided they are within their respective ranges.

Ferulic acid is present in an amount of 0.00050% to 0.01000% by weight relative to the total composition. In a preferred embodiment, ferulic acid is present in an amount of 0.003% to 0.005% by weight of the total composition. In a more preferred embodiment, ferulic acid is present in an amount of 0.001% to 0.002% by weight of the total composition. In an even more preferred embodiment, ferulic acid is present in an amount of 0.00125% by weight relative to the total composition.

Maslinic acid can be present in an amount of 0.001% to 1.0% by weight of the total composition. In a preferred embodiment, maslinic acid may be present in an amount of 0.002% to 0.5% weight of the total composition. In a more preferred embodiment, maslinic acid may be present in an amount of 0.0015% to 0.003% by weight of the total composition. In an even more preferred embodiment, maslinic acid may be present in an amount of 0.002% by weight relative to the total composition.

Olive leaves extract (Olea europaea) may be present in an amount of 0.1% to 5.0% by weight of the total composition. In a preferred embodiment, olive leaves extract is present in an amount of 0.15% to 3.0% by weight of the total composition. In a more preferred embodiment, olive leaves extract is present in an amount of 0.2% to 1.5% by weight of the total composition. In an even more preferred embodiment, olive leaves extract is present in an amount of 0.325% by weight of the total composition.

Octadecyl Di-t-butyl-4-hydroxyhydrocinnamate may be present in an amount of 0.01% to 0.5% by weight of the total composition. In a preferred embodiment, Octadecyl Di-t-butyl-4-hydroxyhydrocinnamate is present in an amount of 0.05% to 0.3% by weight of the total composition. In a more preferred embodiment, Octadecyl Di-t-butyl-4-hydroxyhydrocinnamate is present in an amount of 0.075% to 0.2% by weight of the total composition. In an even more preferred embodiment, Octadecyl Dit-butyl-4-hydroxyhydrocinnamate is present in an amount of 0.1% by weight of the total composition.

Butylated hydroxytoluene (BHT) may be present in an amount of 0.00010% to 0.00030% by weight relative to the total composition. In a preferred embodiment, BHT can be present in an amount of 0.00015% to 0.00030% by weight relative to the total composition. In a more preferred embodiment, BHT can be present in an amount of 0.00020% to 0.00030% by weight relative to the total composition. In an even more preferred embodiment, BHT can be present in an amount of 0.00025% by weight relative to the total composition.

**EXAMPLES**

Described below are examples that illustrate preferred embodiments of the present invention. It should not be understood, however, that they limit the scope of protection of the invention, which is defined solely by the claims accompanying this description.

**Table 1**

| Composition | % mass/mass |
|---|---|
| Sodium Acrylates Copolymer | 1.387500 |

(continued)

| Composition | % mass/mass |
|---|---|
| Lecithin | 0.502500 |
| Xanthan Gum | 0.505000 |
| Disodium EDTA | 0.101500 |
| Ethoxydiglycol | 1.000600 |
| Heptyl Propyl Caprylate / Glyceryl | 3.125500 |
| Coco-caprylate / Caprate | 1.500000 |
| Caprylic Acid Triglyceride / Capric | 1.500000 |
| Propylene / Butylene / Caprylyl Glycol | 9.853500 |
| Methylsilanol Hydroxyproline Aspartate | 0.250000 |
| Dimethylsilanol Hyaluronate | 0.250000 |
| Descarboxy Carnosine HCl | 0.250000 |
| Glutamilamidoetil Imidazole | 0.250000 |
| Acetyl Methionate of Copper / Magnesium / Manganese / Zinc | 1.000000 |
| BHT | 0.000250 |
| Cetyl Palmitate | 0.040000 |
| Cyclomethicone / Dimethicone | 0.035500 |
| Ferulic Acid | 0.001250 |
| Imidazolidinyl Urea | 0.002500 |
| Methylchloroisothiazolinone | 0.000003 |
| Methylisothiazolinone | 0.008316 |
| Polysorbate 80 | 0.001750 |
| Sorbitan Stearate / Oleate | 0.021500 |
| Grape Seed Oil (*Vitis vinifera*) | 0.037500 |
| Glicerin | 0.050000 |
| Sorbitol | 0.050000 |
| Maslinic Acid | 0.002000 |
| Phenoxyethanol | 0.429180 |
| Phenylpropanol | 0.000350 |
| Octadecyl Di-t-butyl-4-hydroxyhydrocinnamate | 0.100000 |
| Fragrance | 0.350000 |
| Propanediol | 0.058688 |
| Olive Leaves Extract (*Olea europaea*) | 0.325000 |
| Water | qsp 100% |

**REVIEWS AND RESULTS**

*MASI Assessment (Melasma Area and Severity Index)*

The MASI assessment consists of precise quantification of the severity of melasma, performed by the visual diagnosis of the face. This is split into four areas: frontal (F), right malar (RM), left malar (LM) and a center (C), corresponding to 30%, 30%, 30% and 10% of the total area, respectively.

Next, the following three parameters are analyzed: Percentage of total area affected (A, grades 0-6, with 6 being representative of 90-100% of the affected area), Hyperpigmentation (D, grades 0-4, with 4 being representative of severe hyperpigmentation) and Homogeneity (H, grades 0-4, with 4 being representative of blemishes that compromise the whole area evaluated).

Finally, the MASI is deliberately calculated by the following equation:

$$MASI = 0.3(DF + HF) \, AF + 0.3(DMR + HMR) \, AMR + 0.3(DML + HML) \, AML + 0.1(DC + HC) \, AC.$$

For the present invention, the MASI analysis was performed before treatment with the composition of Example 1, Initial MASI and after 28 days of use of said composition, and Final MASI. The results are reported in Table 2 below.

**Table 2**

| Participant | Inicial MASI | Final MASI | % Improvement |
|---|---|---|---|
| 1 | 3.8 | 2.6 | 31.6 |
| 2 | 13.7 | 12.5 | 8.8 |
| 3 | 15.0 | 13.8 | 8.0 |
| 4 | 21.9 | 20.4 | 6.8 |
| 5 | 3.1 | 1.2 | 61.3 |
| 6 | 27.5 | 26.4 | 4.0 |
| 7 | 5.8 | 2.6 | 55.2 |
| 8 | 7.4 | 4.4 | 40.5 |
| 9 | 8.4 | 7.2 | 14.3 |
| 10 | 9.9 | 5.2 | 47.5 |
| 11 | 5.4 | 5.4 | 0.0 |
| **Average** | **11.1** | **9.2** | **25.3** |
| **Standard Deviation** | **7.8** | **8.1** | **22.5** |

The results of the analysis were computed using the Wilcoxon paired ranking method with a confidence interval of 95% ($\alpha = 0.05$). From this, it was possible to establish that the composition provides a reduction of melasma intensity between 10.1% and 40.4% with a 95% confidence interval.

Regarding the response to the use of the composition, it was observed that 90.9% of participants responded under the application against 9.1% which did not show improvement after the application time.

*MELASQoL Analysis (Melasma Quality of Life)*

The MELASQoL analysis was formulated based on seven issues of the SKINDEX-16 scale, which evaluates the quality of life of patients with dermatoses in general, being applied to define the impact of melasma on the quality of life of volunteers.

The evaluation consists of applying a questionnaire with ten questions that address different aspects of the lives of the patients. The questionnaire was completed by study participants with the grading on a scale of 1-7 for each item. The final score ranges from 7-70, with the greatest amount corresponding to a lower quality of life.

The results are reported in Table 3 below.

**Table 3**

| Participant | Inicial MELASQoL | Final MELASQoL | % Improvement |
|---|---|---|---|
| 1 | 54.0 | 51.0 | 5.6 |

(continued)

| Participant | Inicial MELASQoL | Final MELASQoL | % Improvement |
|---|---|---|---|
| 2 | 23.0 | 10.0 | 56.5 |
| 3 | 62.0 | 32.0 | 48.4 |
| 4 | 27.0 | 24.0 | 11.1 |
| 5 | 25.0 | 10.0 | 60.0 |
| 6 | 56.0 | 42.0 | 25.0 |
| 7 | 61.0 | 48.0 | 21.3 |
| 8 | 10.0 | 10.0 | 0.0 |
| 9 | 16.0 | 16.0 | 0.0 |
| 10 | 28.0 | 17.0 | 39.3 |
| 11 | 17.0 | 15.0 | 11.8 |
| **Average** | **34.5** | **25.0** | **25.4** |
| **Standard Deviation** | **19.5** | **15.7** | **22.3** |

The results of the analysis were computed using the Wilcoxon paired ranking method with a confidence interval of 95% ($\alpha$ = 0.05). It was noted that 81.8% of participants showed improvement in quality of life against 18.2% who showed no improvement after the exposure time. Although the perception of quality of life improvement is a subjective criterion, there are other parameters that can influence it, and it is believed that the MELASQoL analysis is a good indicator when related to MASI analysis.

From the description and example above, it is possible to observe an unexpected improvement in the composition herein with respect to the prior art.

Although certain embodiments have been specifically described, they were presented only in an exemplary way and are not intended to limit the scope of the invention. The claims accompanying this description and their equivalents are considered to cover such methods.

Finally, modifications of the present invention which are obvious to one skilled in the art, such as adding or removing non-core elements to its implementation, may be made without departing from the scope and spirit of the invention.

**Claims**

1. Composition comprising at least three active compounds selected from ferulic acid, maslinic acid, olive leaves extract and cosmetically acceptable excipients.

2. Composition according to claim 1 comprising at least one additional active compound selected from Octadecyl Di-t-butyl-4-hydroxyhydrocinnamate and BHT.

3. Composition according to claim 1 or 2 **characterized in that** one or more active compounds are encapsulated in carriers selected from the following groups: inclusion complexes, polymeric particles and lipid carriers.

4. Composition according to claim 3 **characterized in that** ferulic acid is encapsulated in carriers selected from the following groups: inclusion complexes, polymeric particles and lipid carriers.

5. Composition according to claim 3 **characterized in that** maslinic acid is encapsulated in one or more carriers selected from the following groups: inclusion complexes, polymeric particles and lipid carriers.

6. Composition according to claim 1 or 2 **characterized in that** ferulic acid is present in an amount of 0.00050% to 0.01000% by weight of the total composition.

7. Composition according to any one of claims 1 to 3, **characterized in that** the Octadecyl Di-t-butyl-4-hydroxyhydrocinnamate is present in an amount of 0.010% to 0.500% by weight of the total composition.

8. Composition according to any one of claims 1 to 4, **characterized in that** the olive leaves extract is present in an amount of 0.100% to 5.000% weight of the total composition.

9. Composition according to any one of claims 1 to 5, **characterized in that** maslinic acid is present in an amount of 0.0010% to 1.0% by weight of the total composition.

10. Composition according to any one of claims 1 to 6, wherein BHT is present in an amount of 0.00010% to 0.00030% by weight of the total composition.

11. Composition according to any one of claims 1 to 7, **characterized by** being in the form of an aerosol, cream, gel, gel-cream, lotion or serum.

12. Composition according to any one of claims 1 to 8, **characterized by** being in the form of a serum.

13. Use of the composition according to any one of claims 1 to 9, **characterized in that** it is an antioxidant formulation with bleaching action.

FIGURE 1

## Sensorial Analysis of the Perceived Efficacy

Europäisches
Patentamt

European
Patent Office

Office européen
des brevets

# EUROPEAN SEARCH REPORT

Application Number

EP 16 15 5094

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | R. Caputo et al.: "NEW TRITERPENES FROM THE LEAVES OF OLEA EUROPAEA", Phytochemistry Phytochemistry, vol. 13 31 December 1974 (1974-12-31), pages 2825-2827, XP002758253, Retrieved from the Internet: URL:http://ac.els-cdn.com/0031942274802499 /1-s2.0-0031942274802499-main.pdf?_tid=a97 fef16-2717-11e6-ad7a-00000aacb35f&acdnat=1 464689506_2b5bcd329a43e84a7cfbe3ff5107e8e7 [retrieved on 2016-05-31] ----- | | INV. A61K8/36 A61Q19/02 A61K8/97 |
| X | DATABASE GNPD [Online] MINTEL; 30 June 2014 (2014-06-30), "Pre-Aesthetix Serum", XP002758254, Database accession no. 2404927 | 1-13 | |
| Y | * the whole document * ----- | 1-13 | TECHNICAL FIELDS SEARCHED (IPC) |
| Y | EP 1 230 926 A1 (NISSHIN OIL MILLS LTD [JP]) 14 August 2002 (2002-08-14) * paragraphs [0001], [0023], [0032], [0143], [0154], [0161] * ----- | 1-13 | A61Q A61K |
| Y | WO 02/13779 A1 (ZYLEPSIS LTD [GB]; CHEETHAM PETER SAMUEL JAMES [GB]) 21 February 2002 (2002-02-21) * page 1, line 29 - line 30 * ----- | 1-13 | |
| Y | US 2007/065396 A1 (MORARIU MARIUS [US]) 22 March 2007 (2007-03-22) * claims 1-4,9,11 * ----- | 1-13 | |
|  | -/-- | | |

The present search report has been drawn up for all claims

| Place of search | Date of completion of the search | Examiner |
|---|---|---|
| Munich | 31 May 2016 | Verrucci, Marinella |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.....................................................................

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 16 15 5094

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2003/133958 A1 (KUNO NORIYASU [JP] ET AL) 17 July 2003 (2003-07-17) * paragraph [0010] * * table 3 * ----- | 1-13 | |
| A | DATABASE GNPD [Online] MINTEL; 31 July 2013 (2013-07-31), "Brillian Cream - Brightening Moisturizing Cream", XP002758255, Database accession no. 2123100 * the whole document * ----- | 1-13 | |

TECHNICAL FIELDS
SEARCHED      (IPC)

The present search report has been drawn up for all claims

| Place of search | Date of completion of the search | Examiner |
|---|---|---|
| Munich | 31 May 2016 | Verrucci, Marinella |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 16 15 5094

This annex lists the patent family members relating to the patent documents cited in the above-mentioned European search report.
The members are as contained in the European Patent Office EDP file on
The European Patent Office is in no way liable for these particulars which are merely given for the purpose of information.

31-05-2016

| Patent document cited in search report | | | Publication date | Patent family member(s) | | | Publication date |
|---|---|---|---|---|---|---|---|
| EP 1230926 | A1 | | 14-08-2002 | AU | 768306 | B2 | 04-12-2003 |
| | | | | AU | 7686600 | A | 23-04-2001 |
| | | | | CA | 2385139 | A1 | 19-04-2001 |
| | | | | CN | 1409637 | A | 09-04-2003 |
| | | | | EP | 1230926 | A1 | 14-08-2002 |
| | | | | JP | 4025546 | B2 | 19-12-2007 |
| | | | | US | 2002176903 | A1 | 28-11-2002 |
| | | | | WO | 0126670 | A1 | 19-04-2001 |
| WO 0213779 | A1 | | 21-02-2002 | AU | 7576401 | A | 25-02-2002 |
| | | | | WO | 0213779 | A1 | 21-02-2002 |
| US 2007065396 | A1 | | 22-03-2007 | US | 2007065396 | A1 | 22-03-2007 |
| | | | | US | 2012114719 | A1 | 10-05-2012 |
| | | | | US | 2014377338 | A1 | 25-12-2014 |
| US 2003133958 | A1 | | 17-07-2003 | AU | 4468201 | A | 08-10-2001 |
| | | | | CA | 2404012 | A1 | 04-10-2001 |
| | | | | CN | 1427707 | A | 02-07-2003 |
| | | | | EP | 1295587 | A1 | 26-03-2003 |
| | | | | JP | 3958968 | B2 | 15-08-2007 |
| | | | | KR | 20030005252 | A | 17-01-2003 |
| | | | | US | 2003133958 | A1 | 17-07-2003 |
| | | | | WO | 0172265 | A1 | 04-10-2001 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008116959 A

### Non-patent literature cited in the description

- **EMERIT, I.** Free Radicals and Aging of the Skin. *Birkhauser Basel,* 1992, vol. 62, 328-341
- **RATHI, S.K. ; ACHAR, A.** Melasma: A Clinical-Epidemiological Study of 312 Cases. *Indian J Dermatol.,* July 2011, vol. 56 (4), 380-382
- **GRIMES, P.E.** Melasma. Etiologic and therapeutic considerations. *Arch Dermatol.,* December 1995, vol. 131 (12), 1453-7
- **MONTEIRO, R.C. et al.** A comparative study of the efficacy of hydroquinone 4% vs 0.75% kojic acid cream in the treatment of facial melasma. *Indian J Dermatol,* 2013, vol. 58, 157
- **LOZANO-MENA et al.** Maslinic acid, a natural phytoalexin-type triterpene from olives. - A promising nutraceutical?. *Molecules,* 2014, vol. 19, 11538